(19) Europäisches Patentamt
European Patent Office
Office européen des brevets

(11) Veröffentlichungsnummer : **0 413 264 B1**

(12) **EUROPÄISCHE PATENTSCHRIFT**

(45) Veröffentlichungstag der Patentschrift :
**12.05.93 Patentblatt 93/19**

(51) Int. Cl.$^5$ : **C07C 53/50,** C07C 51/60

(21) Anmeldenummer : **90115344.5**

(22) Anmeldetag : **10.08.90**

# (54) Verfahren zur Herstellung von Chlorcarbonsäurechloriden.

(30) Priorität : **17.08.89 DE 3927146**

(43) Veröffentlichungstag der Anmeldung :
**20.02.91 Patentblatt 91/08**

(45) Bekanntmachung des Hinweises auf die Patenterteilung :
**12.05.93 Patentblatt 93/19**

(84) Benannte Vertragsstaaten :
**BE CH DE ES FR GB IT LI NL**

(56) Entgegenhaltungen :
**EP-A- 0 050 775**
**EP-A- 0 253 214**

(73) Patentinhaber : **BASF Aktiengesellschaft**
**Carl-Bosch-Strasse 38**
**W-6700 Ludwigshafen (DE)**

(72) Erfinder : **Franzischka, Wolfgang, Dr.**
**Flomersheimer Strasse 36**
**W-6710 Frankenthal (DE)**
Erfinder : **Hupfer, Leopold, Dr.**
**Waltershoehe 3**
**W-6701 Friedelheim (DE)**
Erfinder : **Henkelmann, Jochem, Dr.**
**Volkerstrasse 26**
**W-6140 Bensheim (DE)**
Erfinder : **Kahl, Thomas-Michael, Dr.**
**Germersheimer Strasse 146**
**W-6725 Roemerberg (DE)**
Erfinder : **Sauerwald, Manfred, Dr.**
**Im Langen Satz 2**
**W-6701 Meckenheim (DE)**

**EP 0 413 264 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Chlorcarbonsäurechloriden der Formel I

$$R\text{-}CHCl\text{-}(CH_2)_n\text{-}COCl \qquad (I)$$

in der R Wasserstoff oder einen C-organischen Rest bedeutet und n für einen Wert von 2 bis 4 steht, durch Umsetzung eines Lactons der Formel II

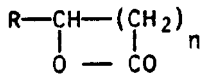 (II)

mit Phosgen in Gegenwart eines Katalysators.

Aus der DE-A 36 24 258 ist die Herstellung von Chlorcarbonsäurechloriden des Typs I durch Umsetzung der entsprechenden Lactone II mit Phosgen in Gegenwart quartärer Ammoniumsalze als Katalysatoren bekannt. Obwohl man nach diesem Verfahren gute Ausbeuten erzielt, läßt es in verfahrenstechnischer Hinsicht zu wünschen übrig, weil die quartären Ammoniumsalze sowohl in den Lactonen als auch in den Chlorcarbonsäurechloriden unlöslich sind, weswegen sich die Aufarbeitung des Reaktionsgemisches und besonders auch eine kontinuierliche Verfahrensführung erschweren.

Der Erfindung lag daher die Aufgabe zugrunde, diesen Nachteilen abzuhelfen.

Demgemäß wurde ein Verfahren zur Herstellung von Chlorcarbonsäurechloriden der Formel I

$$R\text{-}CHCl\text{-}(CH_2)_nCOCl \qquad (I)$$

in der R Wasserstoff oder einen C-organischen Rest bedeutet und n für einen Wert von 2 bis 4 steht, durch Umsetzung eines Lactons der Formel II

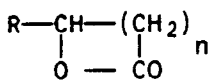 (II)

mit Phosgen in Gegenwart eines Katalysators gefunden, welches dadurch gekennzeichnet ist, daß man die Umsetzung in Gegenwart eines Phosphinoxides (III) als Katalysator vornimmt.

Die Eignung der Phosphinoxide III ist nach den bisherigen Beobachtungen nicht von der Art der organischen Reste abhängig, so dar man grundsätzlich beliebige Verbindungen dieser Art einsetzen kann. Vorzugsweise verwendet man jedoch aliphatische Phosphinoxide wie Trialkylphosphinoxide, deren Alkylreste, die unverzweigt oder verzweigt sein können, 1 bis 12, vorzugsweise 4 bis 8 C-Atome enthalten. Geeignete Verbindungen dieser Art sind z.B. Trihexylphosphinoxid und Tributylphosphinoxid sowie vor allem Trioctylphosphinoxid und Butyl- und But-2-yl-dioctylphosphinoxid.

Weiterhin kommen cycloaliphatische Phosphinoxide wie Tricyclohexylphosphinoxid, araliphatische Phosphinoxide wie Tribenzylphosphinoxid und Phosphinoxide mit gemischten Resten wie Hexyldiphenylphosphinoxid in Betracht. Weniger geeignet sind Arylphosphinoxide. Auch Bisphosphinoxide und cyclische Phosphinoxide der allgemeinen Formeln

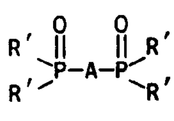 und 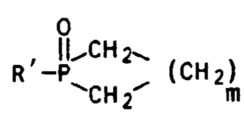 III

in denen A eine Alkylengruppe mit 2 bis 6 Brückengliedern bedeutet und die Reste R' gleiche oder verschiedene organische Reste, beispielsweise die obengenannten Reste, bezeichnen und m für 2 oder 3 steht, kommen in Betracht.

Man verwendet die Phosphinoxide vorzugsweise in einer Menge von 0,1 bis 10, insbesondere 0,5 bis 5 mol-% der Menge des Lactons II.

Als Ausgangsverbindungen II sind im Hinblick auf die Bedeutung der Verfahrensprodukte I vor allem die unsubstituierten Lactone, also das Butyrolacton, das Valerolacton und das Caprolacton zu nennen. Die Substituenten R können grundsätzlich beliebig sein, sofern sie sich unter den Reaktionsbedingungen inert verhalten. In Betracht kommen beispielsweise $C_1$-$C_6$-Alkylgruppen, die Phenylgruppe, die Benzylgruppe und die

Cyclohexylgruppe und hiervon abgeleitete substituierte Reste.

Werden flüssige oder niedrig schmelzende Lactone eingesetzt, so kann die Reaktion in aller Regel ohne Lösungsmittel durchgeführt werden. Ansonsten empfiehlt es sich, die Umsetzung in einem inerten Lösungsmittel vorzunehmen. Als Lösungsmittel eignen sich hochsiedende Kohlenwasserstoffe wie Cumol oder aromatische Chlorverbindungen wie Dichlorbenzol oder auch bevorzugt das Verfahrensprodukt in Mengen von etwa bis zu 50 Gew.%, bezogen auf II.

Es empfiehlt sich, eine Lösung aus dem Lacton und dem Phosphinoxid sowie gegebenenfalls dem Lösungsmittel vorzulegen und das Phosgen gasförmig mit der Geschwindigkeit in diese Lösung einzuleiten, mit der es reagiert. Auf diese Weise kommt man mit 1 bis 3 mol Phosgen pro Mol II aus. Der Phosgenüberschuß wird in üblicher Art und Weise in das Reaktionsgefäß zurückgeführt.

Häufig ist die zusätzliche Mitverwendung von Chlorwasserstoff vorteilhaft, und zwar in Mengen von 5 bis 100, vorzugsweise 20 bis 40 mol-% des Lactons.

Vorzugsweise nimmt man die Umsetzung bei 60 bis 200°C, insbesondere bei 90 bis 180°C vor.

Normalerweise arbeitet man bei Atmosphärendruck. Geringere Drücke, etwa im Bereich von 500 bis 980 mbar, können aus sicherheitstechnischen Gründen zweckmäßig sein, um einen Gasaustritt zu vermeiden, und höhere Drücke, etwa bis 10 bar, können im Falle höherer Temperaturen erforderlich sein, um die Verdampfung von flüchtigen Komponenten zu unterbinden.

Da es sich um eine homogene Reaktion handelt, gestaltet sich die Aufarbeitung des Reaktionsgemisches auf die Verfahrensprodukte I besonders einfach, indem sie auf rein destillative Weise vorgenommen werden kann. Sie bietet methodisch keine Besonderheiten, so daß sich nähere Ausführungen erübrigen.

Die Vorteile des erfindungsgemäßen Verfahrens treten bei kontinuierlicher Arbeitsweise besonders hervor, sei es unter Verwendung einer Rührkesselkaskade oder einer im Gegenstrom betriebenen Reaktionskolonne, da man es in beiden Fällen nicht mehr mit dem verfahrenstechnischen Problem zu tun hat, einen ungelösten Katalysator gleichmäßig im Reaktionsgemisch zu verteilen und zu transportieren. Das kontinuierliche Verfahren läßt sich nach den hierfür üblichen Techniken ausführen, so daß weitere Angaben hierzu entbehrlich sind.

Die Chlorcarbonsäurechloride sind bekanntermaßen wertvolle Zwischenprodukte für organische Synthesen, insbesondere von Pflanzenschutzmitteln und Arzneimitteln.

Beispiele 1 bis 4

In eine Lösung aus 1 mol eines Lactons II (R = H; n variabel) und a mol eines Trialkylphosphinoxids $P(O)Alk_3$ wurde im Laufe von t Stunden bei T°C b mol Phosgen und c mol Chlorwasserstoff eingeleitet.

Nach beendeter Umsetzung wurde das Reaktionsgemisch bei etwa 100°C durch Durchleiten von Stickstoff von überschüssigem Phosgen und Chlorwasserstoff befreit, wonach es destillativ aufgearbeitet wurde. Die Einzelheiten dieser Beispiele sowie deren Ergebnisse sind der folgenden Tabelle zu entnehmen.

Tabelle

| Beispiel | Lacton II | Phosphinoxid Alk/a [mol] | Phosgen b [mol] | HCl c [mol] | T[°C] t [h] | Chlorcarbonsäurechloride Ausbeute | Reinheit | Sdp mbar |
|---|---|---|---|---|---|---|---|---|
| 1 | Butyrolacton | n-Butyl 0,02 | 1,20 | 0,04 | 130-150 5 | 95,6 | 99,2 | 90-91 70 |
| 2 | Butyrolacton | * | 1,08 | 0,04 | 140-150 5 | 91,2 | 99,6 | s.o. |
| 3 | Valerolacton | n-Octyl 0,02 | 1,20 | - | 165-170 7 | 94,8 | 99,6 | 103-105 45 |
| 4 | Caprolacton | n-Butyl 0,03 | 1,20 | - | 165-175 7 | 90,2 | 99,0 | 113-115 30 |

*in Form des Destillationsrückstandes aus Beispiel 1

Beispiel 5

In einem Hauptreaktor von 350 ml Inhalt wurde eine Mischung aus 50 g/h (0,6 mol/h) Butyrolacton und 2 bis 3 mol-% Tri-n-butylphosphinoxid mit 60 g/h (0,6 mol/h) Phosgen und 10 g/h (0,27 mol/h) Chlorwasserstoff bei 140-150°C umgesetzt. Das Reaktionsgemisch ließ man in einem zweiten Reaktor (250 ml) weiterreagieren. Die Aufarbeitung des 4-Chlorbuttersäurechlorids erfolgte analog den Beispielen 1 bis 4.
Kp = 90-91°C/70 mbar;
Ausbeute: 92,4 %; Reinheit: 99,3 %

## Patentansprüche

1. Verfahren zur Herstellung von Chlorcarbonsäurechloriden der Formel I

$$R\text{-}CHCl\text{-}(CH_2)_n\text{-}COCl \qquad (I)$$

in der R Wasserstoff oder einen C-organischen Rest bedeutet und n für einen Wert von 2 bis 4 steht, durch Umsetzung eines Lactons der Formel II

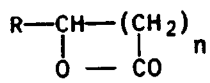

(II)

mit Phosgen in Gegenwart eines Katalysators, dadurch gekennzeichnet, daß man hierbei ein Phosphinoxid (III) als Katalysator verwendet.

2. Verfahren nach Anspruch 1, dadurch gekennzeichnet, daß man die Umsetzung in Gegenwart von Chlorwasserstoff durchführt.

3. Verfahren nach Anspruch 1 oder 2, dadurch gekennzeichnet, daß man die Umsetzung bei 60 bis 200°C vornimmt.

4. Verfahren nach den Ansprüchen 1 bis 3, dadurch gekennzeichnet, daß man als Phosphinoxid ein Trialkylphosphinoxid verwendet, dessen Alkylreste gleich oder verschieden sein können und je 1 bis 12 C-Atome enthalten.

## Claims

1. A process for preparing chloro carbonyl chlorides of the formula I

$$R\text{-}CHCl\text{-}(CH_2)_n\text{-}COCl \qquad (I)$$

where R is hydrogen or a C-organic radical and n is from 2 to 4, by reacting a lactone of the formula II

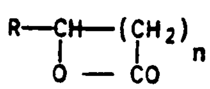

(II)

with phosgene in the presence of a catalyst, wherein a phosphine oxide (III) is used as catalyst for this.

2. A process as claimed in claim 1, wherein the reaction is carried out in the presence of hydrogen chloride.

3. A process as claimed in claim 1 or 2, wherein the reaction is carried out at from 60 to 200°C.

4. A process as claimed in claims 1 to 3, wherein a trialkylphosphine oxide whose alkyl radicals can be identical or different and each contain 1 to 12 carbons is used as phosphine oxide.

## Revendications

1. Procédé de préparation de chlorures d'acides chlorocarboxyliques de formule I

$$R\text{-}CHCl\text{-}(CH_2)_n\text{-}COCl \qquad (I)$$

dans laquelle R représente un atome d'hydrogène ou un reste C-organique et n est mis pour un nombre de 2 à 4, par réaction d'une lactone de formule II

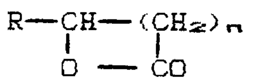

(II)

avec du phosgène en présence d'un catalyseur, caractérisé en ce qu'on utilise, comme catalyseur, un oxyde de phosphine (III).

2. Procédé selon la revendication 1, caractérisé en ce que la réaction est conduite en présence de gaz chlorhydrique.

3. Procédé selon la revendication 1 ou 2, caractérisé en ce qu'on procède à la réaction à une température de 60 à 200°C.

4. Procédé selon l'une quelconque des revendications 1 à 3, caractérisé en ce qu'on utilise, comme oxyde de phosphine, un oxyde de trialkylphosphine dont les restes allyle peuvent être identiques ou différents et contiennent chacun de 1 à 12 atomes de carbone.